# EUROPÄISCHE PATENTSCHRIFT

(11) **EP 1 587 802 B1**
(45) Veröffentlichungstag und Bekanntmachung des Hinweises auf die Patenterteilung: **14.11.2007**
(21) Anmeldenummer: 04702333.8
(22) Anmeldetag: 15.01.2004
(51) Int. Cl.: C07D 333/22

(54) **HERSTELLUNG UND VERWENDUNG VON 3-METHYLAMINO-1-(2-THIENYL)-1-PROPANON**
PRODUCTION AND USE OF 3-METHYLAMINO-1-(2-THIENYL)-1-PROPANONE
PRODUCTION ET UTILISATION DE 3-METHYLAMINO-1-(2-THIENYLE)-1-PROPANONE

(30) Priorität: 22.01.2003 DE 10302595
(43) Veröffentlichungstag der Anmeldung: 26.10.2005
(73) Patentinhaber: BASF Aktiengesellschaft, 67056 Ludwigshafen (DE)
(72) Erfinder: STÜRMER, Rainer, 67127 Rödersheim-Gronau (DE)
(86) Internationale Anmeldenummer: PCT/EP2004/000237
(87) Internationale Veröffentlichungsnummer: WO 2004/065376

(56) Entgegenhaltungen:
- EP-A- 0 457 559
- WO-A-20/04020391
- LIU H ET AL: "CHEMO-ENZYMATIC SYNTHESIS OF THE ANTIDEPRESSANT DULOXETINE AND ITS ENANTIOMER" CHIRALITY, WILEY-LISS, NEW YORK, US, Bd. 12, Nr. 1, 2000, Seiten 26-29, XP009000316 ISSN: 0899-0042 in der Anmeldung erwähnt

## Beschreibung

Die vorliegende Erfindung betrifft die Herstellung und Verwendung von 3-Methylamino-1-(2-thienyl)-1-propanon.

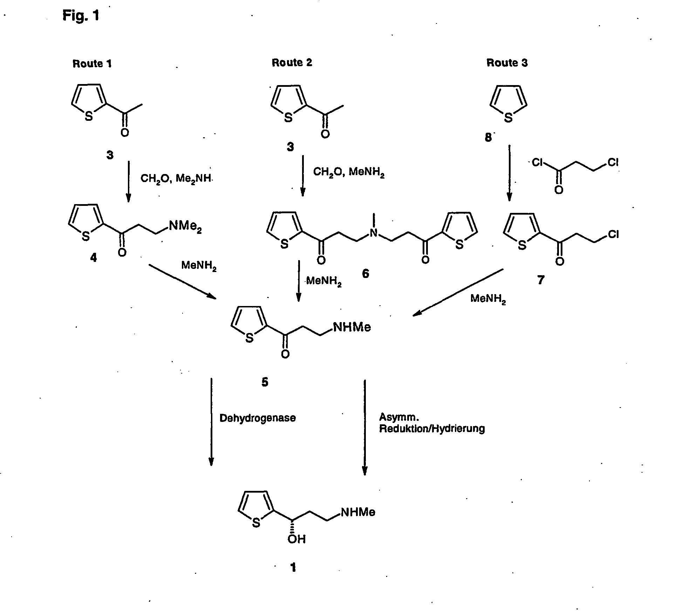

Der Aminoalkohol 1 (Fig.1) [(1S)-3-Methylamino-1-(2-thienyl)-propan-1-ol] ist ein gefragtes Zwischenprodukte bei der Herstellung eines Pharmazeutikums ((+)-(S)-N-methyl-3-(1 -naphthyloxy)-3-(2-thienyl)propylamine oxalat- Handelsname Duloxetine^{®}). Die bisherige Herstellmethode für dieses Intermediat ist aufwendig und benötigt teure und empfindliche Reagenzien. Ferner wird zur Herstellung einer reinen Verbindung eine technisch aufwendige Chromatographie benötigt. Siehe beispielsweise EP 273658 A1; Liu et. al., Chirality 2000, 12 (1), 26-29; Wheeler et.al, J. Labelled Comp. Radiopharm. 1995. 36(3), 213-23; US 5362886, EP 457559, Deeter et al , Tet. Lett. 1990, 31(49). 7101-4; EP0650965; L.A. Sorbera, R.M. Castaner, J. Castaner, Drugs of the Future 2000, 25(9) : 907-916.

WO 2004/020391 und WO 2004/005239 beschreiben die Herstellung von 3-Methylamino-1-(2-thienyl)-1-propanon aus Acetylthiophen, Paraformaldehyd und Methylaminhydrochlorid.

Es bestand daher die Aufgabe, einfachere und kostengünstigere Verfahren zur Herstellung von Duloxetine^{®} zur Verfügung zu stellen.

Die vorliegende Erfindung beschreibt neue kostengünstige Verfahren zu der isomerenreinen Verbindung 1. Die erfindungsgemäßen Verfahren benutzen als gemeinsame Zwischenstufe das Keton 5 (Fig. 1) [3-Methylamino-1-(2-thienyl)-1-propanon] aus dem durch enantioselektive Reduktion der Aminoalkohol 1 gewonnen werden kann. Die weitere Umsetzung des Aminoalkohols 1 zu Duloxetine^{®} ist dem Fachmann geläufig und kann analog zu dem in EP 0457559 A2 beschriebenen Verfahren (Umsetzen mit 1-Fluornaphtalin) durchgeführt werden.

Die Erfindung betrifft die Herstellung von 3-Methylamino-1-(2-thienyl)-1-propanon (Fig. 1, Verbindung 5) sowie seine Säureadditionssalze. Die Säureadditionssalze Verbindung 5 sind Umsetzungsprodukte der Verbindung 5 mit anorganischen oder organischen Säuren. Besonders geeignete Säuren hierfür sind Salzsäure, Schwefelsäure, Phosphorsäure, Oxalsäure, Fumarsäure, Maleinsäure, Essigsäure

Die Herstellung des Keton 5 bzw. des Aminoalkohols 1 kann ausgehend von Thiophen bzw. 2-Acetylthiophen erfolgen. In Fig. 1 sind drei Wege zur Herstellung des Keton 5 dargestellt (Route 1 bis 3), die im folgenden beschrieben werden:

### Route 1

Über eine klassische Mannichreaktion wird ausgehend von Acetylthiophen, Formaldehyd und Dimethylamin Verbindung 4 erhalten (EP 0457559A2 Beispiel 1). Durch Umsetzung von 4 mit einem Überschuss Methylamin wird durch Retro-michael / Michael-reaktion das Monomethylaminoketon 5 erhalten.

### Route 2

Über eine klassische Mannichreaktion wird ausgehend von Acetylthiophen, Formaldehyd und Methylamin Verbindung 6 (Blicke; Burckhalter; JACSAT; J.Amer.Chem.Soc.; 64; 1942; 451, 453.) erhalten .Durch Umsetzung von 6 mit einem Überschuß Methylamin wird durch Retro-michael / Michael-reaktion das Monomethylaminoketon 5 erhalten.

### Route 3 (erfindungsgemäß)

Über eine klassiche Friedel-crafts-acylierung von Thiophen 8 mit 3-chlorpropionsäurechlorid wird die Verbindung 7 (beschrieben bei EI-Khagawa. Ahmed M.; EI-Zohry, Maher F.; Ismail, Mohamed T.; PREEDF; Phosphorus Sulfur; EN; 33; 1987; 25-32) erhalten. Durch Umsetzung mit Methylamin wird das Monomethylaminoketon 5 erhalten.

Ein weiterer Gegenstand der Erfindung ist die Verwendung von 3-Methylamino-1-(2-thienyl)-1-propanon oder seiner Säureadditionssalze zur Herstellung von N-methyl-3-(1-naphthyloxy)-3-(2-thienyl)propylamin oder dessen Säureadditionssalze in racemischer oder enantiomerenreiner Form. Besonders bevorzugt ist die Verwendung zur Herstellung (+)-(S)-N-methyl-3-(1-naphthyloxy)-3-(2-thienyl)propylamin oxalat (Duloxetin^{®}).

Ein weiterer Gegenstand der Erfindung ist ein Verfahren zur Herstellung von N-methyl-3-(1-naphthyloxy)-3-(2-thienyl)propylamin oder seiner Säureadditionssalze in racemischer oder bevorzugt in enantiomerenreiner Form, wobei in einem ersten Schritt 3-Methylamino-1-(2-thienyl)-1-propanon oder seine Säureadditionssalze als Zwischenprodukt hergestellt werden, die anschließend zum entsprechenden Alkohol reduziert werden.
Die Reduktion kann sowohl unter racemisierenden Bedingungen oder enantioselektiv durchgeführt werden. Bevorzugt ist eine enantioselektive Reduktion, insbesondere eine solche, die das (S)-Enantiomer 1 als Produkt liefert.

Dies kann sowohl chemisch mit klassischen enantioselektiven Hydrierverfahren wie beispielsweise NaBH₄ oder LiAIH₄, die zur Erzielung einer Enantioselektivität mit chiralen Liganden versehen sind, durchgeführt werden, oder mittels übergangsmetallhaltiger Hydrierkatalysatoren oder mittels enzymatischer Reduktionen beispielsweise mittels mikrobieller, speziell bakterieller oder pilzlicher Dehydrogenasen.

Ein weiterer Gegenstand der Erfindung ist ein Verfahren zur Herstellung (+)-(S)-N-methyl-3-(1-naphthyloxy)-3-(2-thienyl)propylamin oxalat (Duloxetin^{®}) wobei als Zwischenprodukt 3-Methylamino-1-(2-thienyl)-1-propanon oder ein Säureadditionssalz davon hergestellt wird, das durch Umsetzung von Thiophen mit 3-Chlorpropionsäurechlorid und anschliessende Umsetzung mit Methylamin erhalten wird.

### Experimentelles :

### Route 1 :

5 g Dimethylaminoketon 4 als Hydrochlorid werden in 25 ml Ethanol vorgelegt, tropfenweise mit 20 eq. Methylamin (40 % in Wasser) versetzt und 6 h bei 60-70°C gerührt. Nach Abschluss der Reaktion wird das Ethanol teilweise entfernt und das Produkt 5 als weisser kristalliner Feststoff erhalten (Ausbeute 3.45 g als Hydrochlorid)

### Route 2 :

5 g Diketon 6 als Hydrochlorid werden in 25 ml Ethanol vorgelegt, tropfenweise mit 20 eq. Methylamin (40 % in Wasser) versetzt und 6 h bei 70-80°C gerührt. Nach Abschluss der Reaktion wird das Ethanol teilweise entfernt und das Produkt 5 als weißer kristalliner Feststoff erhalten (Ausbeute 3.87 g als Hydrochlorid) Route 3 (erfindungsgemäß):

5 g Chlorketon 7 werden in 25 ml THF vorgelegt, tropfenweise mit 20 eq. Methylamin (40 % in Wasser) versetzt und 6 h bei 30-40°C gerührt. Nach Abschluss der Reaktion wird das THF größtenteils entfernt und das Produkt 5 als weißer kristalliner Feststoff isoliert (Ausbeute 4.10 g als Hydrochlorid)

Bei den Routen 1-3 kann wässriges Methylamin auch durch gasförmiges oder verflüssigtes Methylamin ersetzt werden.

Spektroskopische Daten des Monomethylaminoketons 5 als Hydrochlorid: ¹³C-NMR(D₂O. 125 MHz) Multiplizitäten aus Spin-Echo in Klammem:
δ (ppm)= 188.5 (s). 140.4 (s), 139.2 (d), 137.8 (d), 131.9 (d), 46.9 (t). 37.3 (t), 36.0 (q) ¹H-NMR (D₂O, 500 MHz):
δ (ppm) = 8.00 (m, 1H), 7.95 (m, 1H), 7.25 (m, 1 H), 3.40 (m, 2H), 2.75 (m, 2H), 2.62 (s, 3H)

### Reduktion von Verbindung 5 zu Verbindung 1 (Fig.1)

### NaBH4 (racemisch):

5 g Methylaminoketon 5 wurden in 20 ml Ethanol vorgelegt und bei 20°C portionsweise mit 0.8 eq. NaBH4 versetzt. Nach 6 h Rühren wurde wässrig aufgearbeitet. Der racemische Monomethylaminoalkohol 1 wurde als schwachgelber Feststoff erhalten (Ausbeute : 3.9 g)
1H-NMR (500 MHz, CDCl3)
δ(ppm)= 2.1 (m, 2H), 2.5 (s, 3H), 2.9 (m, 2H), 4.5 (br s, 2H), 5.25 (m, 1H), 6.94 (m, 1H), 7.00 (m, 1H), 7.22 (m,1H)
13-C-NMR (125 MHz, CDCl3)
δ(ppm) = 35.4, 36.3, 49.7, 71.4, 122.5, 123.8, 126.6, 149.3

LiAIH4 (chiral modifiziert) wie in EP 0457559 A2, example 1B durchgeführt (enantioselektiv).

Die Ausbeute an 1 betrug 74 % mit einer Enantiomerenreinheit von 72 % ee.

## Patentansprüche

1. Verfahren zur Herstellung (+)-(S)-N-methyl-3-(1-naphthyloxy)-3-(2-thienyl)propylamin oxalat (Duloxetin^{®}) wobei als Zwischenprodukt 3-Methylamino-1-(2-thienyl)-1-propanon oder ein Säureadditionssalz davon hergestellt wird, das durch Umsetzung von Thiophen mit 3-Chlorpropionsäurechlorid und anschliessende Umsetzung mit Methylamin erhalten wird.

2. Verfahren nach Anspruch 1 **dadurch gekennzeichnet, daß** 3-Methylamino-1-(2-thienyl)-1-propanon oder ein Säureadditionssalz davon zu (1S)-3-Methylamino-1-(2-thienyl)-propan-1-ol oder einem Säureadditionssalz davon reduziert wird.

3. Verfahren nach Anspruch 2, **dadurch gekennzeichnet, daß** die Reduktion mit einer mikrobiellen Dehydrogenase durchgeführt wird.

## Claims

1. A process for preparing (+)-(S)-N-methyl-3-(1-naphthyloxy)-3-(2-thienyl)propylamine oxalate (Duloxetin^{®}), wherein 3-methylamino-1-(2-thienyl)-1-propanone, or an acid addition salt thereof, which is obtained by reaction of thiophene with 3-chloropropionyl chloride and subsequent reaction with methylamine, is prepared as intermediate.

2. The process according to claim 1, wherein 3-methylamino-1-(2-thienyl)-1-propanone, or an acid addition salt thereof, is reduced to (1S)-3-methylamino-1-(2-thienyl)propan-2-ol, or an acid addition salt thereof.

3. The process according to claim 2, wherein the reduction is carried out using a microbial dehydrogenase.

## Revendications

1. Procédé de préparation d'oxalate de (+)-(S)-N-méthyl-3-(1-naphtyloxy)-3-(2-thiényl)propylamine (Duloxétine^{®}), dans lequel du 3-méthylamino-1-(2-thiényl)-1-prapanone ou un sel d'addition à un acide de celui-ci, qui est obtenu par réaction de thiophène avec du chlorure d'acide 3-chloropropionique et réaction consécutive avec de la méthylamine, est préparé en tant que produit intermédiaire.

2. Procédé selon la revendication 1, **caractérisé en ce que** le 3-méthylamino-1-(2-thiényl)-1-propanone ou un sel d'addition à un acide de celui-ci est réduit pour donner du (1S)-3-méthylamino-1-(2-thiényl)-propan-1-ol ou un sel d'addition à un acide de celui-ci.

3. Procédé selon la revendication 2, **caractérisé en ce que** la réduction est mise en oeuvre avec une déshydrogénase microbienne.
